(19) **Europäisches Patentamt**
**European Patent Office**
**Office européen des brevets**

(11) **EP 2 508 165 A1**

(12) **EUROPEAN PATENT APPLICATION**
published in accordance with Art. 153(4) EPC

(43) Date of publication:
**10.10.2012 Bulletin 2012/41**

(21) Application number: **10834378.1**

(22) Date of filing: **30.11.2010**

(51) Int Cl.:
*A61K 8/49* (2006.01)          *A61K 8/02* (2006.01)
*A61K 8/365* (2006.01)         *A61K 8/73* (2006.01)
*A61K 8/81* (2006.01)          *A61Q 1/00* (2006.01)
*A61Q 11/00* (2006.01)

(86) International application number:
**PCT/JP2010/006985**

(87) International publication number:
**WO 2011/067924 (09.06.2011 Gazette 2011/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2009 JP 2009276727**

(71) Applicant: **Kao Corporation**
**Chuo-ku**
**Tokyo 103-8210 (JP)**

(72) Inventors:
- **SHIDAHARA, Yasuhiro**
**Wakayama-shi**
**Wakayama 640-8580 (JP)**

- **TAKAGI, Michiya**
**Wakayama-shi**
**Wakayama 640-8580 (JP)**
- **FUKUDA, Kimikazu**
**Wakayama-shi**
**Wakayama 640-8580 (JP)**
- **NAKATSU, Susumu**
**Tokyo 131-8501 (JP)**
- **ICHIMURA, Ikuhisa**
**Tokyo 131-8501 (JP)**
- **TOKUNAGA, Tadayuki**
**Tokyo 131-8501 (JP)**

(74) Representative: **HOFFMANN EITLE**
**Patent- und Rechtsanwälte**
**Arabellastrasse 4**
**81925 München (DE)**

(54) **HYDROGEL PARTICLES**

(57)     Hydrogel particles include: (A) a water-insoluble complex containing catechins; (B) a compound which forms chelate together with divalent or trivalent metal ions; (C) a divalent or trivalent metal salt; (D) a gel-forming agent; and (E) water.

**Description**

TECHNICAL FIELD

The present disclosure relates to hydrogel particles, methods for producing hydrogel particles, and dentifrices and cosmetics using hydrogel particles.

BACKGROUND ART

Catechins and tannins, for example, are known as polyphenols contained in tea leaves. In particular, catechins are known to have various physiological functions such as antioxidant activity, antibacterial activity, reduction of blood cholesterol level, suppression of increase in blood pressure, and suppression of increase in blood glucose. Catechins, however, are strong antioxidant substances in themselves, and thus, cause oxidation or polymerization in storage. In addition, catechins disadvantageously show color change after oxidation and/or polymerization, and for this reason, can be applied only to a small range of products. Accordingly, there has been a demand for a method for suppressing oxidation and polymerization of catechins.

To meet the demand, Patent Document 1 proposes a technique in which hydrogel particles containing catechins and specific polymer forming a water-insoluble complex together with catechins are used to remarkably suppress color change in products.

Patent Document 2 shows a technique in which catechins in the form of hydrogel particles are contained in a dentifrice composition. According to Patent Document 2, this technique allows catechins to be held with stability so that color change thereof can be suppressed. In addition, hydrogel particles are easily broken by brushing, thereby enabling catechins to effectively act near the surfaces of teeth and gums. Moreover, a dentifrice composition with excellent feeling in use can be obtained.

Further, Patent Document 3 shows a technique in which active substances such as polyphenols are combined by polymer and contained in hydrogel particles.

CITATION LIST

PATENT DOCUMENT

[Patent Document 1] Japanese Patent Publication No. 2008-24651
[Patent Document 2] Japanese Patent Publication No. 2008-24652
[Patent Document 3] Japanese Patent No. 3972372

SUMMARY OF THE INVENTION

Hydrogel particles according to the present disclosure include: (A) a water-insoluble complex containing catechins; (B) a compound which forms chelate together with divalent or trivalent metal ions; (C) a divalent or trivalent metal salt; (D) a gel-forming agent; and (E) water.

A dentifrice according to the present disclosure includes the hydrogel particles of the present disclosure.

A cosmetic according to the present disclosure includes the hydrogel particles of the present disclosure.

A method for producing hydrogel particles according to the present disclosure includes formation of droplets by dropping, spraying, or stirring a mixture including (A) a water-insoluble complex containing catechins, (B) a compound which forms chelate together with divalent or trivalent metal ions, (C) a divalent or trivalent metal salt, (D) a gel-forming agent, and (E) water.

DESCRIPTION OF EMBODIMENTS

An embodiment will be described hereinafter.

(Hydrogel particles)

Hydrogel particles according to this embodiment include: (A) a water-insoluble complex containing catechins; (B) a compound which forms chelate together with divalent or trivalent metal ions; (C) a divalent or trivalent metal salt, (D) a gel-forming agent, and (E) water.

In the hydrogel particles of this embodiment, (B) the compound which forms chelate together with divalent or trivalent metal ions and (C) the divalent or trivalent metal salt serve as stabilizers for (A) the water-insoluble complex containing catechins.

The "hydrogel particles" herein mean one or more particles in which (A) the water-insoluble complex containing catechins is dispersed in hydrogel. The concept of hydrogel particles herein does not include a capsule composed of an outer layer, i.e., a shell, and an inner layer, i.e., a core, which are concentrically disposed. The "hydrogel" herein is a gel obtained from (D) the gel-forming agent using (E) water as a solvent.

The shape of the hydrogel particles is not specifically limited, and is preferably the shape of a body of revolution composed of a curved surface. The "body of revolution composed of a curved surface" means a shape defined by revolving a closed plane formed by a virtual axis and a continuous curve about the virtual axis, and does not include shapes having flat surfaces, such as triangular pyramids and cylinders. The shape of the hydrogel particles is more preferably spherical from the viewpoint of aesthetic appearance.

The average particle size of the hydrogel particles of this embodiment is preferably 5-10000 $\mu$m, more preferably 30-3000 $\mu$m, and much more preferably 50-1000 $\mu$m. The average particle size of the hydrogel particles can be measured by a laser diffraction/scattering method or a sieve method. In the case of the laser diffraction/scattering method, the median particle size is measured using a particle size distribution analyzer (e.g., LA-920 manufactured by HORIBA, Ltd.), and the obtained size is used as an average particle size. In the case of the sieve method, 100 g of the hydrogel particles are subjected to wet classification in water with meshed screens, redundant moisture is removed with filter paper, and then the mass of the resultant substance is measured so that the weight-average particle size thereof is used as an average particle size.

The volume-based average particle size of the water-insoluble complex in the hydrogel particles of this embodiment is preferably 0.1-50 $\mu$m, more preferably 0.5-35 $\mu$m, and much more preferably 1-20 $\mu$m. The volume-based average particle size of the water-insoluble complex can be measured in a dispersion before particle formation using a laser diffraction/scattering particle size distribution analyzer (e.g., LA-920 manufactured by HORIBA, Ltd.).

[Component (A)]

The component (A) is a water-insoluble complex containing catechins.

This water-insoluble complex containing catechins includes catechins as a component (A1) and polymer A as a component (A2).

The water-insoluble complex containing catechins can be obtained, as a material which is precipitated as an insoluble matter in water, by adding an aqueous solution of (A2) polymer A to an aqueous solution of (A1) catechins, and mixing these solutions with, for example, an agitator. Formation of such a water-insoluble complex can suppress oxidation and/or polymerization of catechins. Precipitation of the water-insoluble complex can be detected based on turbidity of the solution mixture or the presence of a peak derived from the water-insoluble complex observed when the solution mixture was analyzed with the laser diffraction/scattering particle size distribution analyzer. More specifically, the precipitation can be detected by tests which will be described in examples below.

Examples of (A1) catechins include: for example, non-polymer catechins which are non-epi-catechins such as catechin, gallocatechin, catechin gallate, and gallocatechin gallate; and epi-catechins such as epicatechin, epigallocatechin, epicatechin gallate, and epigallocatechin gallate. Catechins may be made of a single species or a plurality of species.

The content of catechins in the hydrogel particles is preferably 0.1-10 mass %, more preferably 0.3-6 mass %, and much more preferably 0.5-3 mass %, from the viewpoints of allowing catechins to sufficiently act on gums in the case of being contained in dentifrices, and stability of the hydrogel particles.

Catechins can be obtained by, for example, concentrating or purifying a tea extract extracted from tea leaves with hot water or a water-soluble organic solvent. The tea extract may be obtained by using a commercially available concentrate of a tea extract such as "Polyphenon" produced by Mitsui Norin Co., Ltd., "Teaflan" produced by Ito En Ltd., and "Sunphenon" produced by Taiyo Kagaku Co., Ltd., and adjusting the components of the concentrate.

The polymer A as the component (A2) is polymer which forms a water-insoluble complex together with catechins.

Examples of the polymer A include polyvinylpyrrolidone (PVP), polyvinyl alcohol (PVA), hydroxyethyl cellulose (HEC), methyl cellulose (MC), hydroxypropyl cellulose (HPC), hydroxypropyl methyl cellulose (HPMC), polyethylene glycol (PEG), polyglycerol fatty acid ester, polyoxyethylene alkyl ether, polyoxyethylene sorbitan alkyl ester (polyoxyethylene sorbitan fatty acid ester), gelatin, and sodium casein. Among these materials, polyvinylpyrrolidone (PVP), hydroxyethyl cellulose (HEC), methyl cellulose (MC), hydroxypropyl cellulose (HPC), and polyethylene glycol (PEG) are preferable, and polyvinylpyrrolidone (PVP) is especially preferable. The polymer A may be made of a single species or a plurality of species.

The content of the polymer A is preferably 1-4 times by mass, more preferably 1-3 times by mass, and much more preferably 1.2-2.2 times by mass, as large as that of catechins from the viewpoint of effectively reducing color change of catechins.

The weight-average molecular weight of the polymer A is preferably 6,000 or more, more preferably 60,000 or more, much more preferably 400,000 or more, and still much more preferably 1,300,000 or more from the viewpoint of effectively suppressing color change of catechins. From the viewpoint of inhibiting agglomeration of the water-insoluble complex and, thereby, obtaining a fine water-insoluble complex, the weight-average molecular weight is preferably 3,000,000 or less, and more preferably 2,000,000 or less. The weight-average molecular weight of the polymer A is a value obtained by, for example, a viscometry or a light-scattering method as a general method for measuring the weight-average molecular weight. In a case where the polymer A is polyvinylpyrrolidone, the weight-average molecular weight is determined by a K-value calculated based on Fikentscher's formula from the obtained viscosity.

[Component (B)]

The component (B) is a compound which forms chelate together with divalent or trivalent metal ions. This chelate agent stabilizes the water-insoluble complex containing catechins, together with a component (C) which will be described later.

Examples of the compound which forms chelate together with divalent or trivalent metal ions include alginic acid and the salt thereof, tripolyphosphoric acid and the salt thereof, silicic acid and the salt thereof, citric acid and the salt thereof, malic acid and the salt thereof, and polyacrylic acid and the salt thereof. Among these materials, alginic acid and the salt thereof, and tripolyphosphoric acid and the salt thereof are especially preferable from the viewpoint of highly effectively stabilizing the water-insoluble complex. From the viewpoint of stabilizing the water-insoluble complex containing catechins, a sodium alginate aqueous solution preferably has a high viscosity. Specifically, Kimica Algin ULV-5 (a 10 mass % aqueous solution, viscosity: 500-600 mPa·s at 20°C), IL-2 (a 1 mass % aqueous solution, viscosity: 20-50 mPa·s at 20°C), I-5 (viscosity: 500-600 mPa·s at 20°C), and I-S (viscosity: 950-1000 mPa·s at 20°C) produced by KIMICA corporation are preferable.

The component (B) of the compound which forms chelate together with divalent or trivalent metal ions may be made of a single species or a plurality of species. Accordingly, the component (B) may be made of a single species or a plurality of species of acids, a single or a plurality of species of salts, or a mixture of these acids and salts.

The content of the component (B) in the hydrogel particles is preferably 0.05-5.0 mass %, more preferably 0.1-3.0 mass %, and much more preferably 0.15-2.0 mass % from the viewpoints of stabilizing the water-insoluble complex containing catechins and reducing an increase in viscosity of the dispersion in producing hydrogel particles.

[Component (C)]

The component (C) is a divalent or trivalent metal salt. This divalent or trivalent metal salt stabilizes the water-insoluble complex containing catechins together with the component (B). The divalent or trivalent metal salt may be in the form of metal ions, or may form chelate together with the component (B), in the hydrogel particles.

Examples of the divalent metal salt include: calcium salts such as calcium chloride and calcium lactate; zinc salts such as zinc chloride, zinc acetate, and zinc sulfate; and copper salts such as copper chloride and copper sulfate. Example of the trivalent metal salt include: iron salts such as iron chloride and iron sulfate; and aluminum salts such as aluminium chloride and aluminum nitrate. Among these materials, calcium lactate is especially preferable from the viewpoints of safety and avoidance of corrosion in production facilities. The metal salt may be made of a single species or a plurality of species. Accordingly, the component (C) may be made of a single species or a plurality of species of divalent metal salts, a single species or a plurality of species of trivalent metal salts, or a mixture of these divalent and trivalent metal salts.

The content of the divalent or trivalent metal salt is preferably 0.05-5.0 mass %, preferably 0.1-3.0 mass %, and much more preferably 0.15-2.0 mass %. The mass ratio of the content of the component (B) to the content of the divalent or trivalent metal salt, i.e., the content of the component (B)/the content of the metal salt, is preferably 0.3-4.5, more preferably 0.4-3, and much more preferably 0.5-2.5.

[Component (D)]

The component (D) is a gel-forming agent.

The gel-forming agent is preferably an agent which forms non-crosslinked hydrogel. The "non-crosslinked hydrogel" herein is a product of gelation caused not by reaction with ions, e.g., potassium ions or calcium ions, but by heat-reversibility of sol-gel as is the case of agar. The dissolution temperature of agars in water is generally 75°C or more. Among the agars, commonly-used agars are dissolved in water at 75-90°C. The gelation temperature of agar when being cooled after dissolution in water is 30-45°C.

Examples of the gel-forming agent include agar, gelatin, and gellan gum. The gel-forming agent may be made of a single species or a plurality of species.

Among the above-listed gel-forming agents, agar is preferable. From the viewpoint of feeling in use, agar having a jelly strength of 68.6 kPa (700 g/cm$^2$) or less is preferable, and agar having a jelly strength of 19.6 kPa (200 g/cm$^2$) to 63.7 kPa (650 g/cm$^2$) is more preferable. This jelly strength is obtained by a Nikkansuishiki method. Specifically, the Nikkansuishiki method is, for example, performed in the following manner. First, a 1.5 mass % aqueous solution of a gel-forming agent is prepared, and is allowed to stand at 20°C for 15 hours to obtain a gel product. Then, a load is applied to the gel using a Nikkansuishiki jelly strength measuring apparatus (manufactured by Kiya-seisakusho Co., Ltd.). The jelly strength is obtained as the maximum mass (g) per a unit surface area (1 cm$^2$) when the gel withstands the load for 20 seconds at 20°C.

The content of the gel-forming agent in the hydrogel particles is preferably 0.1-8.0 mass % and more preferably 0.5-5.0 mass % from the viewpoint of preventing destruction in mixing the hydrogel particles in dentifrices or cosmetics.

[Component (E)]

The component (E) is water. Examples of water include distilled water and ion-exchanged water. The content of water in the hydrogel particles is preferably 80-97 mass %, and more preferably 85-95 mass %.

[Other components]

The hydrogel particles of this embodiment may include other components as necessary, in addition to the component (A), the component (B), the component (C), the component (D), and the component (E).

Examples of the other components mentioned above include oil components such as solid-, semisolid-, or liquid perfume, fats and oils, waxes, hydrocarbons, higher fatty acids, higher alcohols, esters, oil medicinal ingredients, and silicone oils, surfactants such as nonionic surfactants, anionic surfactants, cationic surfactants, and amphoteric surfactants, water-soluble organic compounds and preservatives such as sugars, polyhydric alcohols, and water-soluble polymer compounds, water-soluble coloring agents, and antioxidants.

<Dentifrice>

The hydrogel particles of this embodiment can be included in dentifrices as an ingredient. The content of the hydrogel particles in a dentifrice can be arbitrarily determined as long as functions of catechins can be exhibited. The content of the hydrogel particles may be 100%, and in general, is preferably 0.01-15 mass %, more preferably 0.1-10 mass %, and much more preferably 1-5 mass %.

The base material for the dentifrice may contain a binder. Examples of the binder include sodium alginate, sodium carboxymethyl cellulose, carrageenan, xanthan gum, sodium polyacrylate, hydroxyethyl cellulose, hydroxypropyl cellulose, pectin, tragacanth gum, gum arabic, guar gum, karaya gum, locust bean gum, gellan gum, tamarind gum, psyllium seed gum, polyvinyl alcohol, sodium chondroitin sulfate, and methoxy ethylene-maleic anhydride copolymer. Among these materials, sodium carboxymethyl cellulose, carrageenan, and xanthan gum are especially preferable. The binder may be made of a single species or a plurality of species. From the viewpoints of storage stability, appropriate viscosity of the dentifrice, shape retention, and refreshing feeling, the content of the binder in the base material for the dentifrice is preferably 0.1-3 mass %, more preferably 0.1-2 mass %, and much more preferably 0.2-1.2 mass %.

The base material for the dentifrice may contain water. The content of water in the base material for the dentifrice is preferably 1-50 mass %, and more preferably 3-40 mass % from the viewpoint of storage stability and appropriate refreshing feeling of the dentifrice.

The base material for the dentifrice may contain other ingredients capable of being used for oral use, e.g., an antioxidant, a foaming agent, a polishing agent, a humectant, a sweetening agent, a flavoring agent, a pH adjuster, a preservative, and various medicinal ingredients.

The antioxidant may be an antioxidative or reductive component capable of being used as a composition for use in oral cavity. Examples of this component include L-ascorbic acid and the salt thereof, erythorbic acid and the salt thereof, (α-tocopherol, α-tocopherol acetate (dl-form, d-form)), a rosemary extract, a stevia extract, a sunflower seed extract, propyl gallate, dibutylhydroxytoluene, butylhydroxyanisole, L-cysteine hydrochloride, hydroquinone and the glycoside thereof, nordihydroguaiaretic acid, ascorbic acid higher fatty acid ester (e.g., laurate, stearate, isostearate, palmitate), and guaiacum. Examples of salts of L-ascorbic acid and erythorbic acid include sodium salt, calcium salt, ferrous salt, and the salt of palmitate. The antioxidant may be made of a single species or a plurality of species. The content of the antioxidant in the base material for a dentifrice is preferably 0.0005-5 mass %, more preferably 0.001-2 mass %, and much more preferably 0.01-2 mass % from the viewpoint of inhibiting color change of appearance.

Examples of the foaming agent include anionic surfactants, nonionic surfactants, cationic surfactants, and amphoteric surfactants.

Examples of the pH adjuster include citric acid and the salt thereof, phosphoric acid and the salt thereof, malic acid and the salt thereof, gluconic acid and the salt thereof, maleic acid and the salt thereof, aspartic acid and the salt thereof, succinic acid and the salt thereof, glucuronic acid and the salt thereof, fumaric acid and the salt thereof, glutamic acid and the salt thereof, adipic acid and the salt thereof, hydrochloric acid, and alkali metal hydroxide. The pH of the dentifrice is preferably 5-7, more preferably 5.5-7, and much more preferably 5.5-6.5 from the viewpoint of stability of catechins in the hydrogel particles and flavor. The pH of the dentifrice is measured with respect to its 10 mass % aqueous solution.

The dentifrice may be a paste dentifrice, a liquid dentifrice, or a gelled dentifrice, for example, according to the use thereof.

In normal tooth brushing, i.e., brushing or rubbing with a finger or a hand, using the dentifrice containing the hydrogel particles of this embodiment, the hydrogel particles in the dentifrice are distributed to teeth, gums, and gingival sulci, and are destroyed to be crushed and spread on teeth and gums, especially gingival sulci. This causes the water-insoluble complex containing catechins to be efficiently delivered to the gingival sulci, where the catechins are released from the polymer A. Then, further brushing or rubbing causes the water-insoluble complex to be further spread into periodontal pockets and sweat glands, leading to delivery of catechins to deeper portions of the periodontal pockets and sweat glands. To crush and spread hydrogel particles to distribute the water-insoluble complex to teeth, gums, and gingival sulci and to appropriately release catechins from the polymer A therein, several different physicochemical mechanisms can be employed. Specifically, moisture containing various types of ions at low ionic strengths, especially sodium chloride, and a lipid mixture including phospholipid, is constantly excreted from salivary glands in mouths and sweat gland and sebaceous glands in skins. On the other hand, releasing of catechins from the polymer A is controlled by an equilibrium relationship with surroundings. Accordingly, dilution of the water-insoluble complex with the moisture described above enables catechins to be successively released.

The above-described dentifrice is useful for prevention and improvement of periodontal disease. The average particle size of hydrogel particles contained in this dentifrice is preferably 100-500 $\mu$m and more preferably 140-290 $\mu$m from the viewpoint of allowing the hydrogel particles to reach gingival sulci.

In the case of hydrogel particles contained in the dentifrice, the content of the component (B) (e.g., sodium alginate) in the hydrogel particles is preferably 1.0 mass % or less and more preferably 0.5 mass % or less, and the content of the component (C) (e.g., calcium lactate) is preferably 0.05 mass % or more and more preferably 0.1 mass % or more.

<Cosmetics>

The hydrogel particles of this embodiment can be contained as an ingredient in cosmetics such as antiperspirants, face cleansers, body cleansers, hair shampoos, hair conditioners, and soaps. In cosmetics, the content of the hydrogel particles may be arbitrarily determined as long as functions of catechins are exhibited. From the viewpoint of stability as cosmetics, the content is preferably 0.01-15 mass %, more preferably 0.1-10 mass %, and much more preferably 1-5 mass %.

(Method for producing hydrogel particles)

In a method for producing the hydrogel particles according to this embodiment, a dispersion as a mixture containing (A) the water-insoluble complex containing catechins, (B) the compound which forms chelate together with divalent or trivalent metal ions, (C) the divalent or trivalent metal salt, (D) the gel-forming agent, and (E) water is prepared, and is formed into droplets by dropping, spraying, or stirring the mixture. These droplets may be solidified by cooling.

In a case where hydrogel particles contain (A) the water-insoluble complex containing catechins, this water-insoluble complex heavily agglomerates, and is deposited in fabrication, to be eventually changed into rubber in some cases. Thus, it is difficult to produce such a water-insoluble complex with stability.

However, since the method for producing hydrogel particles of this embodiment uses both (B) the compound which forms chelate together with divalent or trivalent metal ions and (C) the divalent or trivalent metal salt, these components (B) and (C) serve as stabilizers, and allow (A) the water-insoluble complex containing catechins to be dispersed in a solution containing (D) the gel-forming agent without agglomeration and deposition. As a result, the hydrogel particles containing (A) the water-insoluble complex containing catechins can be produced with stability.

The method for producing hydrogel particles of this embodiment may include a step of forming (A) the water-insoluble complex containing catechins from (A1) catechins and (A2) polymer A. In this case, from the viewpoint of stability in producing hydrogel particles, an alkali is preferably added before the step of forming (A) the water-insoluble complex containing catechins is performed.

Examples of the alkali include sodium hydroxide, potassium hydroxide, potassium carbonate, sodium carbonate, sodium tripolyphosphate, and triethanolamine. As the above alkali, a single species of an alkali or a plurality of species of alkalis may be added.

In the method for producing hydrogel particles of this embodiment, (A) the water-insoluble complex containing catechins may be formed by mixing a first liquid containing (A2) the polymer A and a second liquid containing (A1) catechins. In this case, from the viewpoint of stability in producing hydrogel particles, an alkali is also preferably added before formation of (A) the water-insoluble complex containing catechins. The alkali may be added to both or one of the first liquid and the second liquid. However, from the viewpoint of preventing coloring with (A1) catechins, it is preferable that the alkali is previously added only to the first liquid before mixture with the second liquid. In this case, the pH of the first liquid supplemented with the alkali is preferably 6.0-9.0, more preferably 6.5-8.5, and much more preferably 7-8.5.

In the method for producing hydrogel particles of this embodiment, (A) the water-insoluble complex containing catechins is preferably formed at a pH of 5.0-9.0 and more preferably formed at a pH of 5.5-8.5 from the viewpoint of stability in producing the hydrogel particles.

In the method for producing hydrogel particles of this embodiment, it is preferable that one of the first liquid or the second liquid contains (B) the compound which forms chelate together with divalent or trivalent metal ions, and the other liquid contains (C) the divalent or trivalent metal salt. That is, it is preferable that the first liquid contains (B) the compound which forms chelate together with divalent or trivalent metal ions and the second liquid contains (C) the divalent or trivalent metal salt, or that the first liquid contains (C) the divalent or trivalent metal salt and the second liquid contains (B) the compound which forms chelate together with divalent or trivalent metal ions. In addition, (D) the gel-forming agent may be contained in any one of the first liquid or the second liquid. From the viewpoint of preventing coloring with catechin due to thermal hysteresis in dissolving (D) the gel-forming agent, (D) the gel-forming agent is preferably contained in the first liquid.

More preferably, in the method for producing hydrogel particles of this embodiment, the first liquid is prepared by dissolving (A2) the polymer A, (B) the compound which forms chelate together with divalent or trivalent metal ions, and (D) the gel-forming agent in (E) water with heat, the second liquid is prepared by dissolving (A1) catechins and (C) the divalent or trivalent metal salt in (E) water, a dispersion is obtained by adding the second liquid to the first liquid with the first liquid stirred, and this dispersion is formed into droplets with a general dropping, spraying, or stirring technique. An alkali is preferably added to the first liquid before mixture with the second liquid. The pH of the first liquid supplemented with the alkali is preferably 6.0-9.0, more preferably 6.5-8.5, and much more preferably 7-8.5.

The dispersion can be prepared through a stirring and mixing process.

A stirring device for use in this stirring and mixing process is not specifically limited, and may be a known device. Examples of the stirring device include a homomixer, a line mixer, and a disper which exhibit high shearing force. Among them, the homomixer is especially preferable from the viewpoint of operability.

In the stirring and mixing process, from the viewpoint of dispersing the component (A) with stability, the stirring energy applied to the dispersion is preferably 100 kW·min/m$^3$ or more, more preferably 150 kW·min/m$^3$ or more, and much more preferably 200 kW·min/m$^3$ or more. The "stirring energy" herein is obtained by stirring power P (kW)/dispersion volume V (m$^3$) × stirring and mixing time (min). Specific expressions are shown in Japanese Patent Publication No. 2007-161683. For example, an expression for calculating stirring energy in the case of employing a homomixer is as follows:

$$\text{Stirring energy (kW·min/m}^3\text{)}$$

$$= [\text{stirring power P (kW)}] / [\text{dispersion volume V (m}^3\text{)}] \times \text{stirring and mixing time (min)} \quad (I)$$

In the above expression (I), the stirring power P (kW) is calculated from the following empirical formula 1:

$$\text{Stirring power P (kW)} = Np \times n^3 \times d^5 \times \rho / 1000 \qquad \text{(Empirical formula 1)}$$

Np: power number (Np = 1.5 in the case of a homomixer with a stirring tank capacity less than 10 L, and Np = 1.3 in the case of a homomixer with a stirring tank capacity of 10 L or more)

n: stirring rotation number (-/sec)
d: diameter (m) of a stirring impeller
$\rho$: density (kg/m$^3$) of contents (dispersion)

In the stirring and mixing process, the temperature of the dispersion is preferably 60-90°C. From the viewpoint of suppressing degradation of catechins, the pH of the dispersion is preferably 4.5-7.0 (at 80°C).

The viscosity of the dispersion is not specifically limited. Under the temperature at discharge, spraying, or addition in subsequent droplet formation, this viscosity is preferably 10-1000 mPa·s and more preferably 30-800 mPa·s. The viscosity of the dispersion can be measured with a B-type viscometer.

In the droplet formation, the dropping technique is a technique in which a dispersion is discharged through pores and, utilizing properties of the discharged dispersion of changing into droplets due to its surface tension or interfacial tension, these droplets are solidified by cooling in a gas phase such as air or a liquid phase to form hydrogel particles. From the viewpoint of forming hydrogel particles with a uniform particle size, vibration is preferably applied to the dispersion which is being discharged through pores.

In the spraying technique, a spray nozzle is used to discharge (spray) a dispersion therefrom in a gas phase, droplets are formed by utilizing the surface tension of the dispersion, and these droplets are solidified by cooling in the gas phase to form hydrogel particles. In this technique, a continuous-type mixer may be employed to continuously prepare a dispersion so that the dispersion is sprayed from a spray nozzle directly coupled to the continuous-type mixer. In this case, the retention time as a dispersion can be almost completely eliminated, and thus, the dispersion does not always need to be dispersed with stability. Accordingly, even an active ingredient which degrades stability in dispersion can be contained in a large amount.

In the stirring technique, a dispersion is added to a liquid which has the property of not being substantially mixed with the dispersion and is adjusted at a gelation temperature or higher, then shearing force by stirring is applied to the resultant liquid to change the dispersion into fine particles, and utilizing the property of changing into droplets by the interfacial tension, these droplets are solidified by cooling in the liquid which is not substantially mixed with the dispersion, thereby forming hydrogel particles.

In each of these dropping, spraying, and stirring techniques, the temperature of the dispersion at discharge, spraying, or addition is preferably in the range from a gelation temperature to 100°C, both inclusive. Specifically, the temperature of the dispersion is preferably 60-90°C and more preferably 70-80°C. From the viewpoint of easily producing aesthetic spherical hydrogel particles, the dispersion is preferably at a temperature +10°C higher than the gelation temperature or more, and more preferably at a temperature +20°C higher than the gelation temperature or more. The upper limit of the temperature of the dispersion is 100°C, which is the boiling point of water.

Among the dropping technique, the spraying technique, and the stirring technique mentioned above, the spraying technique is especially preferable from the viewpoint of easily producing hydrogel particles.

The hydrogel particles obtained in the manner described above have the same composition as that of the dispersion. Accordingly, the contents of the components in the dispersion are identical to those in the hydrogel particles. The size of the obtained hydrogel particles may be further reduced by pulverization or other processes, as necessary.

EXAMPLES

[Test evaluation 1]

A one mass % aqueous solution was prepared at room temperature for each of 23 types of polymer: polyvinylpyrrolidone (PVP, BASF Japan Ltd., product name: PVP K90, weight-average molecular weight: 1300000); polyvinyl alcohol (PVA, The Nippon Synthetic Chemical Industry Co., Ltd., product name: Gohsenol EG-05); hydroxyethyl cellulose (HEC, Daicel Corporation, product name: HEC Daicel SE600); methyl cellulose (MC, Sigma-Aldrich Japan K.K., product name: methyl cellulose 4000cps); hydroxypropyl cellulose (HPC, Nippon Soda Co., Ltd., product name: HPC-M); hydroxypropyl methyl cellulose (HPMC, Shin-Etsu Chemical Co., Ltd., product name: Metolose 60SH); polyethylene glycol (PEG, Wako Pure Chemical Industries, Ltd., product name: PEG20000); polyglycerol fatty acid ester (Mitsubishi-Kagaku Foods Corporation, product name: Ryoto Polyglycerol L-7D); polyoxyethylene sorbitan fatty acid ester (Kao Corporation, product name: Reodol TW-S120V); sodium casein (KISHIDA CHEMICAL Co., Ltd., product name: sodium casein); gelatin (Nitta Gelatin Inc., product name: #200); xanthan gum (Dainippon Sumitomo Pharma Co., Ltd., product name: Keldent); guar gum (Ina Food Industry Co., Ltd., product name: guar gum CS); locust bean gum (MRC POLYSACCHARIDE Co., Ltd., product name: Soalocust A120); pullulan (HAYASHIBARA CO., LTD., product name: Pullulan); gum arabic (KISHIDA CHEMICAL Co., Ltd., product name: Powdered acacia); carrageenan (MRC POLYSACCHARIDE Co., Ltd., product name: soageena MV320); sodium carboxymethyl cellulose (Daicel Corporation, product name: CMC Daicel 1350); sodium polyacrylate (Nihon Junyaku Co., Ltd., product name: Jurymer AC10S); sucrose fatty acid ester (Mitsubishi-Kagaku Foods Corporation, product name: RYOTO Sugar ester L1595); dextrin (Matsutani Chemical Industry Co., Ltd., product name: Pine-dex #2); pectin (KISHIDA CHEMICAL Co., Ltd., product name: pectin (derived from citrus fruit)); and sodium alkylsulfate (Kao Corporation, product name: Emal 10P). In addition, a 10 mass % aqueous solution of (A1) catechins (produced by Taiyo Kagaku Co., Ltd., product name: Sunphenon 90S) was prepared at room temperature. Then, the aqueous solution of catechins (10 g) and each of the 23 types of polymer solutions (each 90 g) were mixed with a stirrer, and turbidity and precipitation of insoluble matters were observed.

Table 1 shows the results.

[Table 1]

| Material | Manufacturer or distributor /product name | Casein interaction |
|---|---|---|
| polyvinylpyrrolidone (PVP) | BASF Japan Ltd./PVP K90 | turbid |
| polyvinyl alcohol (PVA) | The Nippon Synthetic Chemical Industry Co., Ltd./Gohsenol EG-05 | turbid, partially paste |
| hydroxyethyl cellulose (HEC) | Daicel Corporation/HEC Daicel SE600 | changed into rubber |
| methyl cellulose (MC) | Sigma-Aldrich Japan K.K. /methyl cellulose 4000cps | changed into rubber |
| hydroxypropyl cellulose (HPC) | Nippon Soda Co., Ltd./ HPC-M | changed into rubber |
| hydroxypropyl methyl cellulose (HPMC) | Shin-Etsu Chemical Co., Ltd. /Metolose60SH | changed into rubber |
| polyethylene glycol (PEG) | Wako Pure Chemical Industries, Ltd./PEG20000 | turbid→paste |
| polyglycerol fatty acid ester | Mitsubishi-Kagaku Foods Corporation/ RYOTO polyglycerol ester L-7D | turbid→paste |
| polyoxyethylene sorbitan fatty acid ester | Kao Corporation/ Reodol TW-S120V | turbid, partially paste |
| sodium casein | KISHIDA CHEMICAL Co., Ltd./sodium casein | changed into rubber |
| gelatin | Nitta Gelatin Inc./#200 | turbid, partially paste |
| xanthan gum | Dainippon Sumitomo Pharma Co., Ltd./ Keldent | no reaction |
| guar gum | Ina Food Industry Co., Ltd. /guar gum CS | no reaction |
| locust bean gum | MRC POLYSACCHARIDE Co., Ltd./ Soalocust A120 | no reaction |
| pullulan | HAYASHIBARA CO., LTD. /Pullulan | no reaction |
| gum arabic | KISHIDA CHEMICAL Co., Ltd./powdered acacia | no reaction |
| carrageenan | MRC POLYSACCHARIDE Co., Ltd./ Soageena MV320 | no reaction |
| sodium carboxymethyl cellulose (CMC-Na) | Daicel Corporation/ CMC Daicel 1350 | no reaction |
| sodium polyacrylate | Nihon Junyaku Co., Ltd. /Jurymer AC10S | no reaction |
| sucrose fatty acid ester | Mitsubishi-Kagaku Foods Corporation/ RYOTO Sugar ester L1595 | no reaction |
| dextrin | Matsutani Chemical Industry Co., Ltd./Pine-dex#2 | no reaction |
| pectin | KISHIDA CHEMICAL Co., Ltd./pectin (derived from citrus fruit) | no reaction |
| sodium alkylsulfate | Kao Corporation/Emal 10P | no reaction |

Polyvinylpyrrolidone became turbid. Polyvinyl alcohol, polyoxyethylene sorbitan fatty acid ester, and gelatin became turbid, and were partially changed into paste. Hydroxyethyl cellulose, methyl cellulose, hydroxypropyl cellulose, hydroxypropyl methyl cellulose, and sodium casein were changed into rubber. Polyethylene glycol and polyglycerol fatty acid ester became turbid, and were changed into paste. It is suggested that these turbid or paste-form precipitates are a water-insoluble complex formed by polymer with catechins.

On the other hand, no reactions occurred with respect to xanthan gum, guar gum, locust bean gum, pullulan, gum arabic, carrageenan, sodium carboxymethyl cellulose, sodium polyacrylate, sucrose fatty acid ester, dextrin, pectin, and sodium alkylsulfate. This results suggest that these types of polymer do not form water-insoluble complexes with catechins.

[Test evaluation 2]

(Formation of hydrogel particles)

Hydrogel particles of examples 1-13 and Comparative Examples 1-4 were formed. Tables 2 and 3 show the compositions of these hydrogel particles.

<Example 1>

First, (A2) polyvinylpyrrolidone 1 (BASF Japan Ltd., product name: PVP K30, weight-average molecular weight: 60000), (A2) polyvinylpyrrolidone 2 (BASF Japan Ltd., product name: PVP K90, weight-average molecular weight: 1300000), (B) sodium alginate (KIMICA corporation, product name: Kimica Algin ULV-5), (D) agar 1 (Ina Food Industry Co., Ltd., product name: UP-37), ethyl p-hydroxybenzoate (API Corporation, product name: ethyl p-hydroxybenzoate) as a preservative, and titanium oxide (Tayca Corporation, product name: JA-C) as a coloring agent were dissolved in (E) ion-exchanged water by heating at 90°C, thereby preparing a first liquid. The contents of the components were adjusted in the following manner. The content of the first liquid was 89.67 mass % of the hydrogel particles. In the hydrogel particles, the content of (A2) polyvinylpyrrolidone 1 was 0.40 mass %, the content of (A2) polyvinylpyrrolidone 2 was 1.20 mass %, the content of (B) sodium alginate was 0.30 mass %, the content of (D) agar 1 was 2.00 mass %, the content of ethyl p-hydroxybenzoate was 0.10 mass %, and the content of titanium oxide was 0.50 mass %.

In addition, (A1) catechins (Taiyo Kagaku Co., Ltd., product name: Sunphenon 90S), (C) calcium lactate (Showa Kako Corporation, product name: calcium lactate hydrate), and sodium ascorbate (BASF Japan Ltd., product name: sodium ascorbate) as an antioxidant were dissolved in (E) ion-exchanged water at room temperature (25°C), thereby preparing a second liquid. The contents of the components were adjusted in the following manner. The content of the second liquid was 10 mass % of the hydrogel particles. In the hydrogel particles, the content of (A1) catechins was 1.00 mass %, the content of (C) calcium lactate was 0.30 mass %, and the content of sodium ascorbate was 0.20 mass %.

The second liquid was mixed in the first liquid, and a 1N sodium hydroxide aqueous solution as a third liquid was added to the mixture such that the content of the sodium hydroxide aqueous solution was 0.33 mass % of the hydrogel particles. In this manner, a total amount of 800 g of a mixture liquid was obtained.

Thereafter, while being kept at 80°C, the mixture liquid was stirred and mixed by a homomixer (PRIMIX Corporation, product name: T. K. Robomix, power number Np = 1.5, stirring impeller diameter d = 0.025 m) with a stirring impeller rotated at a stirring rotation number n = 15000 rpm in a stirring and mixing time t = 1.5 minutes, thereby preparing a dispersion. The content (the dispersion) had a density $\rho$ = 1000 kg/m$^3$ and a volume V = 0.00080 m$^3$. Accordingly, the stirring energy was 429 kW·min/m$^3$.

Then, the obtained dispersion was solidified by cooling by spraying droplets into cooling air (at 15°C) with a one-fluid spray nozzle having an orifice diameter of 0.8 mm at a flow rate of 30 L/hr under a spray pressure of 1.5-2.0 MPa (gage pressure), thereby forming hydrogel particles of Example 1. In the hydrogel particles of Example 1, the mass ratio of the component (B) to the component (C) was 1.00.

<Example 2>

Hydrogel particles of Example 2 were formed in the same manner as those of Example 1 except that polyvinylpyrrolidones 1 and 2 as the component (A2) were replaced with hydroxyethyl cellulose (Daicel Corporation, product name: HEC Daicel SE600) whose content in the hydrogel particles was 1.60 mass %. In the hydrogel particles of Example 2, the mass ratio of the component (B) to the component (C) was 1.00.

<Example 3>

Hydrogel particles of Example 3 were formed in the same manner as those of Example 1 except that polyvinylpyrrolidones 1 and 2 as the component (A2) were replaced with polyvinyl alcohol (The Nippon Synthetic Chemical Industry Co., Ltd., product name: Gohsenol EG-05) whose content in the hydrogel particles was 1.60 mass %. In the hydrogel particles of Example 3, the mass ratio of the component (B) to the component (C) was 1.00.

<Example 4>

Hydrogel particles of Example 4 were formed in the same manner as those of Example 1 except that polyvinylpyrrolidones 1 and 2 as the component (A2) were replaced with polyethylene glycol (Wako Pure Chemical Industries, Ltd., product name: PEG20000) whose content in the hydrogel particles was 1.60 mass %. In the hydrogel particles of Example 4, the mass ratio of the component (B) to the component (C) was 1.00.

<Example 5>

Hydrogel particles of Example 5 were formed in the same manner as those of Example 1 except that polyvinylpyrrolidones 1 and 2 as the component (A2) were replaced with polyglycerol fatty acid ester (Mitsubishi-Kagaku Foods Corporation, product name: RYOTO polyglycerol ester) whose content in the hydrogel particles was 1.60 mass %. In the hydrogel particles of Example 5, the mass ratio of the component (B) to the component (C) was 1.00.

<Example 6>

Hydrogel particles of Example 6 were formed in the same manner as those of Example 1 except that divalent calcium lactate as the component (C) was replaced with trivalent aluminium nitrate (KISHIDA CHEMICAL Co., Ltd., product name: aluminum nitrate nonahydrate). In the hydrogel particles of Example 6, the mass ratio of the component (B) to the component (C) was 1.00.

<Example 7>

Hydrogel particles of Example 7 were formed in the same manner as those of Example 1 except that sodium alginate as the component (B) was replaced with sodium tripolyphosphate (NIPPON CHEMICAL INDUSTRIAL CO., LTD., product name: sodium tripolyphosphate) whose content in the hydrogel particles was 0.60 mass %, the content of calcium lactate in the hydrogel particles was 0.60 mass %, and the 1N sodium hydroxide aqueous solution as the third liquid was not mixed. In the hydrogel particles of Example 7, the mass ratio of the component (B) to the component (C) was 1.00.

<Example 8>

Hydrogel particles of Example 8 were formed in the same manner as those of Example 1 except that sodium alginate as the component (B) was replaced with a sodium silicate solution (Wako Pure Chemical Industries, Ltd., product name: sodium silicate solution) whose content in the hydrogel particles was 0.60 mass % (net content of sodium silicate: 0.33 mass %), the content of calcium lactate in the hydrogel particles was 0.60 mass %, and the 1N sodium hydroxide aqueous solution as the third liquid was not mixed. In the hydrogel particles of Example 8, the mass ratio of the component (B) to the component (C) was 0.55.

<Example 9>

Hydrogel particles of Example 9 were formed in the same manner as those of Example 1 except that sodium alginate as the component (B) was replaced with a sodium citrate (IWATA CHEMICAL CO., LTD., product name: trisodium citrate) whose content in the hydrogel particles was 1.00 mass % and the content of calcium lactate in the hydrogel particles was 0.50 mass %. In the hydrogel particles of Example 9, the mass ratio of the component (B) to the component (C) was 2.00.

<Example 10>

Hydrogel particles of Example 10 were formed in the same manner as those of Example 1 except that sodium alginate as the component (B) was replaced with sodium malate (FUSO CHEMICAL CO., LTD., product name: sodium malate) whose content in the hydrogel particles was 1.00 mass % and the content of calcium lactate in the hydrogel particles was 0.50 mass %. In the hydrogel particles of Example 10, the mass ratio of the component (B) to the component (C) was 2.00.

<Example 11 >

Hydrogel particles of Example 11 were formed in the same manner as those of Example 1 except that sodium alginate as the component (B) was replaced with a sodium polyacrylate solution (Nihon Junyaku Co., Ltd., product name: Jurymer AC-103) whose content in the hydrogel particles was 1.25 mass % (net content of sodium polyacrylate: 0.50 mass %). In the hydrogel particles of Example 11, the mass ratio of the component (B) to the component (C) was 1.67.

<Example 12>

Hydrogel particles of Example 12 were formed in the same manner as those of Example 1 except that agar 2 (Ina Food Industry Co., Ltd., product name: AX-200) whose content in the hydrogel particles was 1.50 mass % was further included as the component (D). In the hydrogel particles of Example 12, the mass ratio of the component (B) to the component (C) was 1.00.

<Example 13>

Hydrogel particles of Example 13 were formed in the same manner as those of Example 12 except that the stirring impeller of the homomixer was rotated at a stirring rotation number n = 8000 rpm in a stirring and mixing time t = 2.0 minutes. In the hydrogel particles of Example 13, the mass ratio of the component (B) to the component (C) was 1.00. The stirring energy was 87kW·min/m$^3$.

<Comparative Example 1>

Hydrogel particles of Comparative Example 1 were formed in the same manner as those of Example 1 except that (B) sodium alginate and (C) calcium lactate were not included.

<Comparative Example 2>

Hydrogel particles of Comparative Example 2 were formed in the same manner as those of Example 1 except that (C) calcium lactate was not included.

<Comparative Example 3>

Hydrogel particles of Comparative Example 3 were formed in the same manner as those of Example 1 except that (B) sodium alginate was not included.

<Comparative Example 4>

Hydrogel particles of Comparative Example 4 were formed in the same manner as those of Example 1 except that (C) divalent calcium lactate was replaced with (C') univalent sodium chloride (KISHIDA CHEMICAL Co., Ltd., product name: sodium chloride).
[Table 2]

| | Component | Material | Manufacturer or distributor/ product name | Example | | | | | | | | | | | | |
|---|---|---|---|---|---|---|---|---|---|---|---|---|---|---|---|---|
| | | | | 1 | 2 | 3 | 4 | 5 | 6 | 7 | 8 | 9 | 10 | 11 | 12 | 13 |
| First liquid | A2 | Polyvinyl pyrrolidone 1 | BASF Japan Ltd./PVP K30 | 0.40 | | | | | 0.40 | 0.40 | 0.40 | 0.40 | 0.40 | 0.40 | 0.40 | 0.40 |
| | A2 | Polyvinyl pyrrolidone 2 | BASF Japan Ltd./PVP K90 | 1.20 | | | | | 1.20 | 1.20 | 1.20 | 1.20 | 1.20 | 1.20 | 1.20 | 1.20 |
| | A2 | hydroxyethyl cellulose | Daicel Corporation/ HEC Daicel SE600 | | 1.60 | | | | | | | | | | | |
| | A2 | polyvinyl alcohol | The Nippon Synthetic Chemical Industry Co., Ltd./Gohsenol EG-05 | | | 1.60 | | | | | | | | | | |
| | A2 | polyethylene glycol | Wako Pure Chemical Industries, Ltd./ PEG20000 | | | | 1.60 | | | | | | | | | |
| | A2 | polyglycerol fatty acid ester | Mitsubishi-Kagaku Foods Corporation/ RYOTO polyglycerin ester | | | | | 1.60 | | | | | | | | |

(continued)

| | Component | Material | Manufacturer or distributor/ product name | Example | | | | | | | | | | | | |
|---|---|---|---|---|---|---|---|---|---|---|---|---|---|---|---|---|
| | | | | 1 | 2 | 3 | 4 | 5 | 6 | 7 | 8 | 9 | 10 | 11 | 12 | 13 |
| | B | sodium alginate | KIMICA corporation/ Kimica Algin ULV-5 | 0.30 | 0.30 | 0.30 | 0.30 | 0.30 | 0.30 | | | | | | 0.30 | 0.30 |
| | B | sodium tripolyphosphate | NIPPON CHEMICAL INDUSTRIA L CO., LTD./ sodium tripolyphosph ate | | | | | | | 0.60 | | | | | | |
| | B | sodium silicate | Wako Pure Chemical Industries, Ltd./ sodium silicate solution | | | | | | | | 0.33 | | | | | |
| | B | sodium citrate | IWATA CHEMICAL CO., LTD./ trisodium citrate | | | | | | | | | 1.00 | | | | |
| | B | sodium malate | FUSO CHEMICAL CO., LTD./ sodium malate | | | | | | | | | | 1.00 | | | |

(continued)

| | Component | Material | Manufacturer or distributor/ product name | Example | | | | | | | | | | | | |
|---|---|---|---|---|---|---|---|---|---|---|---|---|---|---|---|---|
| | | | | 1 | 2 | 3 | 4 | 5 | 6 | 7 | 8 | 9 | 10 | 11 | 12 | 13 |
| | B | sodium polyacrylate | Nihon Junyaku Co., Ltd./ Jurymer AC-103 | | | | | | | | | | | 0.50 | | |
| | D | agar 1 | Ina Food Industry Co., Ltd./UP-37 | 2.00 | 2.00 | 2.00 | 2.00 | 2.00 | 2.00 | 2.00 | 2.00 | 2.00 | 2.00 | 2.00 | 2.00 | 2.00 |
| | D | agar 2 | Ina Food Industry Co., Ltd./AX-200 | | | | | | | | | | | | 1.50 | 1.50 |
| | E | ion-exchanged water | - | 85.17 | 85.17 | 85.17 | 85.17 | 85.17 | 85.17 | 79.90 | 80.17 | 84.47 | 84.47 | 84.97 | 83.67 | 83.67 |
| | preservative | ethylphydroxybenzoate | API Corporation/ethyl phydroxybenzoate | 0.10 | 0.10 | 0.10 | 0.10 | 0.10 | 0.10 | 0.10 | 0.10 | 0.10 | 0.10 | 0.10 | 0.10 | 0.10 |
| | coloring agent | titanium oxide | Tayca Corporation/JA-C | 0.50 | 0.50 | 0.50 | 0.50 | 0.50 | 0.50 | 0.50 | 0.50 | 0.50 | 0.50 | 0.50 | 0.50 | 0.50 |

EP 2 508 165 A1

(continued)

| | Component | Material | Manufacturer or distributor/ product name | Example 1 | 2 | 3 | 4 | 5 | 6 | 7 | 8 | 9 | 10 | 11 | 12 | 13 |
|---|---|---|---|---|---|---|---|---|---|---|---|---|---|---|---|---|
| Second liquid | A1 | catechins | Taiyo Kagaku Co., Ltd./ Sunphen on 90S | 1.00 | 1.00 | 1.00 | 1.00 | 1.00 | 1.00 | 1.00 | 1.00 | 1.00 | 1.00 | 1.00 | 1.00 | 1.00 |
| | C | calcium lactate | Showa Kako Corporation/calcium lactate hydrate | 0.30 | 0.30 | 0.30 | 0.30 | 0.30 | | 0.60 | 0.60 | 0.50 | 0.50 | 0.30 | 0.30 | 0.30 |
| | C | aluminium nitrate | KISHIDA CHEMICAL Co., Ltd./ aluminum nitrate nonahydrate | | | | | | 0.30 | | | | | | | |
| | C' | sodium chloride | KISHIDA CHEMICAL Co.,Ltd./ sodium chloride | | | | | | | | | | | | | |
| | E | ion-exchanged water | - | 8.50 | 8.50 | 8.50 | 8.50 | 8.50 | 8.50 | 13.50 | 13.50 | 8.30 | 8.30 | 8.50 | 8.50 | 8.50 |
| | antioxidant | sodium ascorbate | BASF Japan Ltd./sodium ascorbate | 0.20 | 0.20 | 0.20 | 0.20 | 0.20 | 0.20 | 0.20 | 0.20 | 0.20 | 0.20 | 0.20 | 0.20 | 0.20 |
| Third liquid | pH adjuster | 1N-NaOHaq | - | 0.33 | 0.33 | 0.33 | 0.33 | 0.33 | 0.33 | | | 0.33 | 0.33 | 0.33 | 0.33 | 0.33 |
| | | Total | | 100 | 100 | 100 | 100 | 100 | 100 | 100 | 100 | 100 | 100 | 100 | 100 | 100 |

(continued)

| Component | Material | Manufacturer or distributor/ product name | Example | | | | | | | | | | | | |
|---|---|---|---|---|---|---|---|---|---|---|---|---|---|---|---|
| | | | 1 | 2 | 3 | 4 | 5 | 6 | 7 | 8 | 9 | 10 | 11 | 12 | 13 |
| | B/C | | 1.00 | 1.00 | 1.00 | 1.00 | 1.00 | 1.00 | 1.00 | 0.55 | 2.00 | 2.00 | 1.67 | 1.00 | 1.00 |
| | Homomixer rotation number (rpm) | | 15000 | 15000 | 15000 | 15000 | 15000 | 15000 | 15000 | 15000 | 15000 | 15000 | 15000 | 15000 | 8000 |
| | Stirring and mixing time (min) | | 1.5 | 1.5 | 1.5 | 1.5 | 1.5 | 1.5 | 1.5 | 1.5 | 1.5 | 1.5 | 1.5 | 1.5 | 2.0 |
| | Stirring energy (kW·min/m³) | | 429 | 429 | 429 | 429 | 429 | 429 | 429 | 429 | 429 | 429 | 429 | 429 | 87 |
| | Dispersion stability | | A | A | A-B | A | A | A | A | A-B | A-B | A-B | A-B | A | A-B |
| | Dispersion viscosity (mPa·s, 80°C) | | 75 | 100 | 80 | 65 | 70 | 80 | 35 | 400 | 60 | 60 | 55 | 175 | 450 |
| | Hydrogel particle size (μm) | | 140 | 142 | 138 | 135 | 144 | 150 | 140 | 180 | 138 | 140 | 136 | 155 | 190 |

[Table 3]

| Liquid | Component | Material | Manufacturer or distributor/product name | Comparative Example | | | |
|---|---|---|---|---|---|---|---|
| | | | | 1 | 2 | 3 | 4 |
| First liquid | A2 | Polyvinylpyrrolidone 1 | BASF Japan Ltd./PVP K30 | 0.40 | 0.40 | 0.40 | 0.40 |
| | A2 | polyvinylpyrrolidone 2 | BASF Japan Ltd./PVP K90 | 1.20 | 1.20 | 1.20 | 1.20 |
| | A2 | hydroxyethyl cellulose | Daicel Corporation/HEC Daicel SE600 | | | | |
| | A2 | polyvinyl alcohol | The Nippon Synthetic Chemical Industry Co., Ltd./ Gohsenol EG-05 | | | | |
| | A2 | polyethylene glycol | Wako Pure Chemical Industries, Ltd./PEG20000 | | | | |
| | A2 | polyglycerol fatty acid ester | Mitsubishi-Kagaku Foods Corporation/ RYOTO polyglycerin ester | | | | |
| | B | sodium alginate | KIMICA corporation/ Kimica Algin ULV-5 | | 0.30 | | 0.30 |
| | B | sodium tripolyphosphate | NIPPON CHEMICAL INDUSTRIAL CO., LTD./ sodium tripolyphosphate | | | | |
| | B | sodium silicate | Wako Pure Chemical Industries, Ltd./ sodium silicate solution | | | | |
| | B | sodium citrate | IWATA CHEMICAL CO., LTD./trisodium citrate | | | | |
| | B | sodium malate | FUSO CHEMICAL CO., LTD./sodium malate | | | | |
| | B | sodium polyacrylate | Nihon Junyaku Co., Ltd./Jurymer AC-103 | | | | |
| | D | agar 1 | Ina Food Industry Co., Ltd./UP-37 | 2.00 | 2.00 | 2.00 | 2.00 |

(continued)

| Liquid | Component | Material | Manufacturer or distributor/product name | Comparative Example | | | |
|---|---|---|---|---|---|---|---|
| | | | | 1 | 2 | 3 | 4 |
| | D | agar 2 | Ina Food Industry Co., Ltd./AX-200 | | | | |
| | E | ion-exchanged water | - | 85.47 | 85.17 | 85.47 | 85.17 |
| | preservative | ethyl phydroxybenzoate | API Corporation/ ethyl phydroxybenzoate | 0.10 | 0.10 | 0.10 | 0.10 |
| | coloring agent | titanium oxide | Tayca Corporation/JA-C | 0.50 | 0.50 | 0.50 | 0.50 |
| Second liquid | A1 | catechins | Taiyo Kagaku Co., Ltd./Sunphenon 90S | 1.00 | 1.00 | 1.00 | 1.00 |
| | C | calcium lactate | Showa Kako Corporation/ calcium lactate hydrate | | | 0.30 | |
| | C | aluminium nitrate | KISHIDA CHEMICAL Co., Ltd./aluminum nitrate nonahydrate | | | | |
| | C' | sodium chloride | KISHIDA CHEMICAL Co., Ltd./sodium chloride | | | | 0.30 |
| | E | ion-exchanged water | - | 8.80 | 8.80 | 8.50 | 8.50 |
| | antioxidant | sodium ascorbate | BASF Japan Ltd./ sodium ascorbate | 0.20 | 0.20 | 0.20 | 0.20 |
| Third liquid | pH adjuster | 1N-NaOHaq | - | 0.33 | 0.33 | 0.33 | 0.33 |
| Total | | | | 100 | 100 | 100 | 100 |
| B/C | | | | - | - | - | - |
| Homomixer rotation number (rpm) | | | | 15000 | 15000 | 15000 | 15000 |
| Stirring and mixing time (min) | | | | 1.5 | 1.5 | 1.5 | 1.5 |
| Stirring energy (kW·min/ m³) | | | | 429 | 429 | 429 | 429 |
| Dispersion stability | | | | C | C | C | C |
| Dispersion viscosity (mPa·s, 80°C) | | | | 60 | 95 | 60 | 90 |
| Hydrogel particle size (μm) | | | | 135 | 141 | 138 | 144 |

(Test evaluation method)

<Stability of dispersion>

The dispersions before formation of the hydrogel particles of Examples 1-13 and Comparative Examples 1-4 were left at 80°C, and the states of the dispersions after 24 hours were observed. Then, visual inspection was made to categorize the dispersions into A: the water-insoluble complex was hardly precipitated, B: the water-insoluble complex was partially precipitated, but was not changed into rubber, and C: the water-insoluble complex was precipitated and changed into rubber.

<Viscosity of dispersion>

For the dispersions before formation of the hydrogel particles of Examples 1-13 and Comparative Examples 1-4, viscosity at 80°C was measured with a B-type viscometer. In this measurement, a rotor No. 2 was used at a rotation speed of 60 rpm, and the value after one minute was taken.

<Average particle size of hydrogel particles>

Median particle sizes of the hydrogel particles of Examples 1-12 and Comparative Examples 1-4 were measured with a laser diffraction/scattering particle size distribution analyzer (LA-920 manufactured by HORIBA, Ltd.) as average particle sizes of the hydrogel particles.

(Test evaluation results)

Tables 2 and 3 show the test results.

The dispersion stability was A for Example 1, A for Example 2, A-B for Example 3, A for Example 4, A for Example 5, A for Example 6, A for Example 7, A-B for Example 8, A-B for Example 9, A-B for Example 10, A-B for Example 11, A for Example 12, A-B for Example 13, C for Comparative Example 1, C for Comparative Example 2, C for Comparative Example 3, and C for Comparative Example 4. Specifically, substantially no precipitates were observed in Examples 1, 2, 4, 5, 6, 7, and 12 even after 24 hours. In Examples 3, 8, 9, 10, and 13, a small amount of agglomeration was observed after 24 hours. In Example 11, precipitates were observed after 24 hours, but were not changed into rubber and could be dispersed again by shaking. In Comparative Examples 1-4, a water-insoluble substance was gradually deposited, and this deposited substance agglomerated to be finally changed into a rubber-like cluster. This cluster was not dispersed again even after stirring and mixing with a homomixer.

The viscosity of the dispersion was 75 mPa·s for Example 1, 100 mPa·s for Example 2, 80 mPa·s for Example 3, 65 mPa·s for Example 4, 70 mPa·s for Example 5, 80 mPa·s for Example 6, 35 mPa·s for Example 7, 400 mPa·s for Example 8, 60 mPa·s for Example 9, 60 mPa·s for Example 10, 55 mPa·s for Example 11, 175 mPa·s for Example 12, 450 mPa·s for Example 13, 60 mPa·s for Comparative Example 1, 95 mPa·s for Comparative Example 2, 60 mPa·s for Comparative Example 3, and 90 mPa·s for Comparative Example 4.

Comparison between Example 12 and Example 13 with respect to the viscosity of the dispersion shows that the dispersion of Example 12 prepared with higher stirring energy had a lower viscosity and higher stability than the dispersion of Example 13 prepared with lower stirring energy. Microscopic observation demonstrates that dispersion of Example 12 showed uniform dispersion of the water-insoluble complex whereas the dispersion of Example 13 showed partial agglomeration of the water-insoluble complex.

The average particle size of the hydrogel particles was 140 μm for Example 1, 142 μm for Example 2, 138 μm for Example 3, 135 μm for Example 4, 144 μm for Example 5, 150 μm for Example 6, 140 μm for Example 7, 180 μm for Example 8, 138 μm for Example 9, 140 μm for Example 10, 136 μm for Example 11, 155 μm for Example 12, 190 μm for Example 13, 135 μm for Comparative Example 1, 141 μm for Comparative Example 2, 138 μm for Comparative Example 3, and 144 μm for Comparative Example 4.

[Test evaluation3]

(Hydrogel particles)

Hydrogel particles 1-7 were formed as follows. The compositions thereof are also shown in Table 4.

<Hydrogel particles 1>

In the manner similar to that in Example 1 of test evaluation 2, hydrogel particles 1 including 1.0 mass % of (A1) tea catechin (Taiyo Kagaku Co., Ltd., product name: Sunphenon 90S), 0.4 mass % of (A2) polyvinylpyrrolidone 1 (BASF Japan Ltd., product name: PVP K30, weight-average molecular weight: 60000), 1.2 mass % of (A2) polyvinylpyrrolidone 2 (BASF Japan Ltd., product name: PVP K90, weight-average molecular weight: 1300000), 2.0 mass % of (D) agar (Ina Food Industry Co., Ltd., product name: UP-37), 1.0 mass % of ascorbic acid (BASF Japan Ltd.), and 0.5 mass % of titanium oxide (Tayca Corporation, product name: JA-C), with the balance of (E) purified water, and containing an appropriate amount of sodium hydroxide, were formed.

<Hydrogel particles 2>

Hydrogel particles 2 having the same composition as that of the hydrogel particles 1 except that the content of ascorbic acid was 0.5 mass % and 0.1 mass % of ethyl p-hydroxybenzoate (API Corporation, product name: ethyl p-hydroxybenzoate) was further included, were formed.

<Hydrogel particles 3>

Hydrogel particles 3 having the same composition as that of the hydrogel particles 2 except that ascorbic acid was replaced with 0.2 mass % of sodium ascorbate (BASF Japan Ltd.), 0.5 mass % of (B) sodium alginate 1 (KIMICA corporation, product name: Kimica Algin ULV-5), and 0.3 mass % of (C) calcium lactate (Showa Kako Corporation, product name: calcium lactate hydrate), were formed. In the hydrogel particles 3, the mass ratio of the component (B) to the component (C) was 1.67.

<Hydrogel particles 4>

Hydrogel particles 4 having the same composition as that of the hydrogel particles 3 except that the content of (B) sodium alginate 1 was 0.3 mass %, were formed. In the hydrogel particles 4, the mass ratio of the component (B) to the component (C) was 1.00.

<Hydrogel particles 5>

Hydrogel particles 5 having the same composition as that of the hydrogel particles 4 except that the content of (C) calcium lactate was 0.2 mass %, were formed. In the hydrogel particles 5, the mass ratio of the component (B) to the component (C) was 1.50.

<hydrogel particles 6>

Hydrogel particles 6 having the same composition as those of the hydrogel particles 4 and 5 except that sodium alginate 1 as the component (B) was replaced with sodium alginate 2 (KIMICA corporation, product name: Kimica Algin IL-2) and the content of (C) calcium lactate was 0.08 mass %, were formed. In the hydrogel particles 6, the mass ratio of the component (B) to the component (C) was 3.75.

<Hydrogel particles 7>

Hydrogel particles 7 having the same composition as that of the hydrogel particles 6 except that the content of (C) calcium lactate was 0.3 mass %, were formed. In the hydrogel particles 7, the mass ratio of the component (B) to the component (C) was 1.00.
[Table 4]

| Component | Material | Manufacturer or distributor/product name | Hydrogel particles | | | | | | |
|---|---|---|---|---|---|---|---|---|---|
| | | | 1 | 2 | 3 | 4 | 5 | 6 | 7 |
| A1 | tea catechin | Taiyo Kagaku Co., Ltd./Sunphenon 90S | 1.0 | 1.0 | 1.0 | 1.0 | 1.0 | 1.0 | 1.0 |
| A2 | polyvinyl pyrrolidone 1 | BASF Japan Ltd. /PVP K30 | 0.4 | 0.4 | 0.4 | 0.4 | 0.4 | 0.4 | 0.4 |
| A2 | polyvinyl pyrrolidone 2 | BASF Japan Ltd. /PVP K90 | 1.2 | 1.2 | 1.2 | 1.2 | 1.2 | 1.2 | 1.2 |
| B | sodium alginate 1 | KIMICA corporation/ Kimica Algin ULV-5 | | | 0.5 | 0.3 | 0.3 | | |
| B | sodium alginate 2 | KIMICA corporation/ Kimica Algin IL-2 | | | | | | 0.3 | 0.3 |
| C | calcium lactate | Showa Kako Corporation/calcium lactate hydrate | | | 0.3 | 0.3 | 0.2 | 0.08 | 0.3 |
| D | agar | Ina Food Industry Co., Ltd./UP-37 | 2.0 | 2.0 | 2.0 | 2.0 | 2.0 | 2.0 | 2.0 |
| E | purified water | - | balance | balance | balance | balance | balance | balance | balance |
| antioxidant | ascorbic acid | BASF Japan Ltd. /ascorbic acid | 1.0 | 0.5 | | | | | |
| antioxidant | sodium ascorbate | BASF Japan Ltd. /sodium ascorbate | | | 0.2 | 0.2 | 0.2 | 0.2 | 0.2 |
| preservative | ethyl phydroxybenzoate | API Corporation/ ethyl phydroxybenzoate | | 0.1 | 0.1 | 0.1 | 0.1 | 0.1 | 0.1 |
| coloring agent | titanium oxide | Tayca Corporation/JA-C | 0.5 | 0.5 | 0.5 | 0.5 | 0.5 | 0.5 | 0.5 |
| pH adjuster | sodium hydroxide | - | appropriate amount | appropriate amount | appropriate amount | appropriate amount | appropriate amount | appropriate amount | appropriate amount |

(continued)

| Component | Material | Manufacturer or distributor/product name | Hydrogel particles | | | | | | |
|---|---|---|---|---|---|---|---|---|---|
| | | | 1 | 2 | 3 | 4 | 5 | 6 | 7 |
| | Total | | 100 | 100 | 100 | 100 | 100 | 100 | 100 |
| | B/C | | - | - | 1.67 | 1.00 | 1.50 | 3.75 | 1.00 |

(Dentifrice compositions)

Dentifrice compositions 1-7 were formed in the following manner. Compositions thereof are also shown in Table 5.

<Dentifrice composition 1>

A dentifrice composition 1 containing 34 mass % of a sorbitol solution (a 70 mass % aqueous solution), 4 mass % of glycerin (100 mass %), 0.7 mass % of sodium monofluorophosphate, 32 mass % of erythritol (Mitsubishi-Kagaku Foods Corporation, product name: erythritol), 8 mass % of abrasive silica (PQ Corporation, product name: Sorbosil AC77), 4 mass % of thickening silica (Fuji Silysia Chemical Ltd., product name: Silopure 25), 1.2 mass % of sodium lauryl sulfate, 0.4 mass % of sodium carboxymethyl cellulose, 0.2 mass % of carrageenan, 0.1 mass % of xanthan gum, 1 mass % of perfume, 0.1 mass % of DL-malic acid, 0.05 mass % of saccharin sodium, 0.3 mass % of sodium polyphosphate, and 4 mass % of the hydrogel particles 1, with a balance of purified water, was formed.

<Dentifrice composition 2>

A dentifrice composition 2 having the same composition as that of the dentifrice composition 1 except that the content of the sorbitol solution was 35 mass %, the content of erythritol was 30 mass %, the content of abrasive silica was 10 mass %, and the hydrogel particles 1 were replaced with the hydrogel particles 2, was formed.

<Dentifrice composition 3>

A dentifrice composition 3 having the same composition as that of the dentifrice composition 2 except that the content of the sorbitol solution was 33 mass % and the hydrogel particles 2 were replaced with the hydrogel particles 3, was formed.

<Dentifrice composition 4>

A dentifrice composition 4 having the same composition as that of the dentifrice composition 2 except that the content of the sorbitol solution was 34 mass %, the content of thickening silica was 3 mass %, and the hydrogel particles 2 were replaced with the hydrogel particles 4, was formed.

<Dentifrice composition 5>

A dentifrice composition 5 having the same composition as that of the dentifrice composition 2 except that the hydrogel particles 2 were replaced with the hydrogel particles 5, was formed.

<Dentifrice composition 6>

A dentifrice composition 6 having the same composition as that of the dentifrice composition 2 except that the hydrogel particles 2 were replaced with the hydrogel particles 6, was formed.

<Dentifrice composition 7>

A dentifrice composition 7 having the same composition as that of the dentifrice composition 2 except that the hydrogel particles 2 were replaced with the hydrogel particles 7, was formed.

(Test evaluation method)

Immediately after formation of each of the dentifrice compositions 1-7, the composition was placed in a transparent case (PS CASE No.1 manufactured by AS ONE Corporation) with a size of 3 cm × 3 cm × 1 cm, and was photographed on a white board. Then, L*, a*, and b* (hereinafter referred to as "L, a, and b") on a site to be measured in the obtained image were quantified with an ADOBE PHOTOSHOP (registered trademark).

After storage for one month at 50°C, L, a, and b on the site to be measured were quantified in the same manner. The above quantifications were conducted under the same photography conditions for lighting, shutter speed, stop, and focal length.

Thereafter, the difference (ΔE) between L, a, and b immediately after formation (initial stage) and L, a, and b after storage was obtained as the degree of discoloration from the following expression (II):

[Expression 1]

$$X = (([L \text{ in initial stage}] - [L \text{ after storage}])^2 +$$

$$([a \text{ in initial stage}] - [a \text{ after storage}])^2 +$$

$$([b \text{ in initial stage}] - [b \text{ after storage}])^2)$$

$$\text{The degree of discoloration } (\Delta E) = \sqrt{X} \qquad \text{(II)}$$

(Test evaluation results)

Table 5 shows the test results.

[Table 5]

| Component | Dentifrice composition | | | | | | |
|---|---|---|---|---|---|---|---|
| | 1 | 2 | 3 | 4 | 5 | 6 | 7 |
| sorbitol solution (70 mass % aqueous solution) | 34 | 35 | 33 | 34 | 35 | 35 | 35 |
| glycerin | 4 | 4 | 4 | 4 | 4 | 4 | 4 |
| sodium monofluorophosphate | 0.7 | 0.7 | 0.7 | 0.7 | 0.7 | 0.7 | 0.7 |
| erythritol | 32 | 30 | 30 | 30 | 30 | 30 | 30 |
| abrasive silica | 8 | 10 | 10 | 10 | 10 | 10 | 10 |
| thickening silica | 4 | 4 | 4 | 3 | 4 | 4 | 4 |
| sodium lauryl sulfate | 1.2 | 1.2 | 1.2 | 1.2 | 1.2 | 1.2 | 1.2 |
| sodium carboxymethyl cellulose | 0.4 | 0.4 | 0.4 | 0.4 | 0.4 | 0.4 | 0.4 |
| carrageenan | 0.2 | 0.2 | 0.2 | 0.2 | 0.2 | 0.2 | 0.2 |
| xanthan gum | 0.1 | 0.1 | 0.1 | 0.1 | 0.1 | 0.1 | 0.1 |
| perfume | 1 | 1 | 1 | 1 | 1 | 1 | 1 |
| DL-malic acid | 0.1 | 0.1 | 0.1 | 0.1 | 0.1 | 0.1 | 0.1 |
| saccharin sodium | 0.05 | 0.05 | 0.05 | 0.05 | 0.05 | 0.05 | 0.05 |
| sodium polyphosphate | 0.3 | 0.3 | 0.3 | 0.3 | 0.3 | 0.3 | 0.3 |
| hydrogel particles 1 | 4 | | | | | | |
| hydrogel particles 2 | | 4 | | | | | |
| hydrogel particles 3 | | | 4 | | | | |
| hydrogel particles 4 | | | | 4 | | | |

(continued)

| Component | Dentifrice composition | | | | | | |
| --- | --- | --- | --- | --- | --- | --- | --- |
| | 1 | 2 | 3 | 4 | 5 | 6 | 7 |
| hydrogel particles 5 | | | | | 4 | | |
| hydrogel particles 6 | | | | | | 4 | |
| hydrogel particles 7 | | | | | | | 4 |
| purified water | balance | balance | balance | balance | balance | balance | balance |
| Total | 100 | 100 | 100 | 100 | 100 | 100 | 100 |
| Degree of discoloration | 12.0 | 9.8 | 6.7 | 4.8 | 5.7 | 6.5 | 4.0 |

The degree of discoloration was 12.0 for the dentifrice composition 1, 9.8 for the dentifrice composition 2, 6.7 for the dentifrice composition 3, 4.8 for the dentifrice composition 4, 5.7 for the dentifrice composition 5, 6.5 for the dentifrice composition 6, and 4.0 for the dentifrice composition 7.

The dentifrice composition 3-7 including the hydrogel particles 3-7 have lower degrees of discoloration than the dentifrice compositions 1 and 2 respectively including hydrogel particles 1 and 2 containing neither sodium alginate nor calcium lactate. The dentifrice compositions 4, 5, and 7 respectively including hydrogel particles 4, 5, and 7 in which the content of sodium alginate is 0.3 mass % or less and the content of calcium lactate is 0.1 mass % or more significantly reduce discoloration.

[Test evaluation 4]

(Hydrogel particles)

Hydrogel particles of Examples 14 and 15 were formed in the following manner. The compositions of these particles are also shown in Table 6.

<Example 14>

First, (A2) polyvinylpyrrolidone 1, (A2) polyvinylpyrrolidone 2, (B) sodium alginate, (D) agar 1, (D) agar 2, ethyl p-hydroxybenzoate as a preservative, and titanium oxide as a coloring agent were dissolved in (E) ion-exchanged water by heating at 90°C, thereby preparing a first liquid. The contents of the components were adjusted in the following manner. The content of the first liquid was 89.67 mass % of the hydrogel particles. In the hydrogel particles, the content of (A2) polyvinylpyrrolidone 1 was 0.40 mass %, the content of (A2) polyvinylpyrrolidone 2 was 1.20 mass %, the content of (B) sodium alginate was 0.30 mass %, the content of (D) agar 1 was 2.00 mass %, the content of (D) agar 2 was 1.50 mass %, the content of ethyl p-hydroxybenzoate was 0.10 mass %, and the content of titanium oxide was 0.50 mass %.

In addition, (A1) catechins, (C) calcium lactate, and sodium ascorbate as an antioxidant were dissolved in (E) ion-exchanged water at room temperature (25°C), thereby preparing a second liquid. The contents of the components were adjusted in the following manner. The content of the second liquid was 10 mass % of the hydrogel particles. In the hydrogel particles, the content of (A1) catechins was 1.00 mass %, the content of (C) calcium lactate was 0.30 mass %, and the content of sodium ascorbate was 0.20 mass %.

A 1N sodium hydroxide aqueous solution as a third liquid was mixed in the first liquid such that the content of the sodium hydroxide aqueous solution was 0.33 mass % of the hydrogel particles, thereby adjusting the pH to 7.5. Then, the second liquid was mixed in the resultant liquid , thereby obtaining a total amount of 800 g of a mixture liquid. As described above, the alkaline sodium hydroxide was previously added to the first liquid before mixture with the second liquid to adjust the pH to 7.5, and under this pH, the first liquid and the second liquid were mixed together. In this manner, (A) a water-insoluble complex containing catechins was formed from (A1) catechins, (A2) polyvinylpyrrolidone 1, and (A2) polyvinylpyrrolidone 2.

Thereafter, while being kept at 80°C, the mixture liquid was stirred and mixed by a homomixer with a stirring impeller rotated at a stirring rotation number n = 15000 rpm in a stirring and mixing time t = 1.5 minutes, thereby preparing a dispersion. The content (the dispersion) had a density $\rho$ = 1000 kg/m$^3$ and a volume V = 0.00080 m$^3$. Accordingly, the stirring energy was 429 kW·min/m$^3$.

Then, the obtained dispersion was solidified by cooling by spraying droplets into cooling air (at 15°C) with a one-fluid spray nozzle having an orifice diameter of 0.8 mm at a flow rate of 30 L/hr under a spray pressure of 1.5-2.0 MPa (gage pressure), thereby forming hydrogel particles of Example 14. In the hydrogel particles of Example 14, the mass ratio of the component (B) to the component (C) was 1.00.

<Example 15>

First, (A2) polyvinylpyrrolidone 1, (A2) polyvinylpyrrolidone 2, (B) sodium tripolyphosphate, (D) agar 1, (D) agar 2, ethyl p-hydroxybenzoate as a preservative, and titanium oxide as a coloring agent were dissolved in (E) ion-exchanged water by heating at 90°C, thereby preparing a first liquid. The contents of the components were adjusted in the following manner. The content of the first liquid was 79.40 mass % of the hydrogel particles. In the hydrogel particles, the content of (A2) polyvinylpyrrolidone 1 was 0.40 mass %, the content of (A2) polyvinylpyrrolidone 2 was 1.20 mass %, the content of (B) sodium tripolyphosphate was 0.60 mass %, the content of (D) agar 1 was 2.00 mass %, the content of (D) agar 2 was 1.50 mass %, the content of ethyl p-hydroxybenzoate was 0.10 mass %, and the content of titanium oxide was 0.50 mass %. The pH of the first liquid was 8.3.

In addition, (A1) catechins, (C) calcium lactate, and sodium ascorbate as an antioxidant were dissolved in (E) ion-exchanged water at room temperature (25°C), thereby preparing a second liquid. The contents of the components were adjusted in the following manner. The content of the second liquid was 20.60 mass % of the hydrogel particles. In the hydrogel particles, the content of (A1) catechins was 1.00 mass %, the content of (C) calcium lactate was 0.90 mass %, and the content of sodium ascorbate was 0.20 mass %.

A second liquid was mixed in the first liquid, thereby obtaining a total amount of 800 g of a mixture liquid. As described above, the alkaline (B) sodium tripolyphosphate was previously added to the first liquid before mixture with the second liquid to adjust the pH to 8.3, and under this pH, the first liquid and the second liquid were mixed together. In this manner, (A) a water-insoluble complex containing catechins was formed from (A1) catechins, (A2) polyvinylpyrrolidone 1, and (A2) polyvinylpyrrolidone 2.

Thereafter, while being kept at 80°C, the mixture liquid was stirred and mixed by a homomixer with a stirring impeller rotated at a stirring rotation number n = 15000 rpm in a stirring and mixing time t = 1.5 minutes, thereby preparing a dispersion. The content (the dispersion) had a density $\rho$ = 1000 kg/m$^3$ and a volume V = 0.00080 m$^3$. Accordingly, the stirring energy was 429 kW·min/m$^3$.

Then, the obtained dispersion was solidified by cooling by spraying droplets into cooling air (at 15°C) with a one-fluid spray nozzle having an orifice diameter of 0.8 mm at a flow rate of 30 L/hr under a spray pressure of 1.5-2.0 MPa (gage pressure), thereby forming hydrogel particles of Example 15. In the hydrogel particles of Example 15, the mass ratio of the component (B) to the component (C) was 0.67.

[Table 6]

| Liquid | Component | Material | Manufacturer or distributor/ product name | Example | |
|---|---|---|---|---|---|
| | | | | 14 | 15 |
| First liquid | A2 | Polyvinylpyrrolid one 1 | BASF Japan Ltd./PVP K30 | 0.40 | 0.40 |
| | A2 | polyvinylpyrrolid one 2 | BASF Japan Ltd./PVP K90 | 1.20 | 1.20 |
| | B | sodium alginate | KIMICA corporation/ Kimica Algin ULV-5 | 0.30 | |
| | B | sodium tripolyphosphate | NIPPON CHEMICAL INDUSTRIAL CO., LTD./ sodium tripolyphosphate | | 0.60 |
| | D | agar 1 | Ina Food Industry Co., Ltd./ UP-37 | 2.00 | 2.00 |
| | D | agar 2 | Ina Food Industry Co., Ltd./ AX-200 | 1.50 | 1.50 |
| | E | ion-exchanged water | - | 83.67 | 73.10 |
| | preservative | ethyl phydroxybenzoate | API Corporation/ethyl phydroxyb enzo ate | 0.10 | 0.10 |
| | coloring agent | titanium oxide | Tayca Corporation/JA-C | 0.50 | 0.50 |

(continued)

| Liquid | Component | Material | Manufacturer or distributor/ product name | Example | |
|---|---|---|---|---|---|
| | | | | 14 | 15 |
| Second liquid | A1 | catechins | Taiyo Kagaku Co., Ltd./ Sunphenon 90S | 1.00 | 1.00 |
| | c | calcium lactate | Showa Kako Corporation/ calcium lactate hydrate | 0.30 | 0.90 |
| | E | ion-exchanged water | - | 8.50 | 18.50 |
| | antioxidant | sodium ascorbate | BASF Japan Ltd./ sodium ascorbate | 0.20 | 0.20 |
| Third liquid | pH adjuster | 1N-NaOHaq | - | 0.33 | |
| Total | | | | 100 | 100 |
| B/C | | | | 1.00 | 0.67 |
| Homomixer rotation number (rpm) | | | | 15000 | 15000 |
| Stirring and mixing time (min) | | | | 1.5 | 1.5 |
| Stirring energy (kW·min/ m$^3$) | | | | 429 | 429 |

(Stability in forming hydrogel particles)

The formation of Example 12 employs the technique of mixing the first liquid and the second liquid together and then adding the alkaline sodium hydroxide as the third liquid. On the other hand, the formation of Example 14 employs the technique of previously adding the alkaline sodium hydroxide as the third liquid to the first liquid to adjust the pH to 7.5 and then mixing the second liquid into the resultant liquid. As a result, as compared to the dispersion obtained in Example 12, the resultant dispersion of Example 14 showed further reduced precipitation of the water-insoluble complex. Accordingly, it was confirmed that hydrogel particles can be formed with stability from the dispersion of Example 14.

The Example 15 also employs the technique of previously adding the alkaline (B) sodium tripolyphosphate to the first liquid to adjust the pH to 8.3 and then adding the second liquid to the first liquid. As a result, as compared to the dispersion obtained in Example 12, the resultant dispersion of Example 15 showed further reduced precipitation of the water-insoluble complex. Accordingly, it was confirmed that hydrogel particles can be formed with stability from the dispersion of Example 15.

INDUSTRIAL APPLICABILITY

The present disclosure is useful for hydrogel particles, methods for producing hydrogel particles, and dentifrices and cosmetics using hydrogel particles.

**Claims**

1. Hydrogel particles, comprising:

   (A) a water-insoluble complex containing catechins;
   (B) a compound which forms chelate together with divalent or trivalent metal ions;
   (C) a divalent or trivalent metal salt;
   (D) a gel-forming agent; and
   (E) water.

2. The hydrogel particles of claim 1, wherein
   the component (B) is of at least one selected from the group consisting of alginic acid and a salt thereof, tripolyphosphoric acid and a salt thereof, silicic acid and a salt thereof, citric acid and a salt thereof, malic acid and a salt thereof, and polyacrylic acid and a salt thereof.

3. The hydrogel particles of claim 1 or 2, wherein a content of the component (B) is 0.05-5.0 mass %.

4. The hydrogel particles of any one of claims 1-3, wherein the component (C) is of at least one selected from the group consisting of calcium salt, zinc salt, copper salt, iron salt, and aluminum salt.

5. The hydrogel particles of any one of claims 1-4, wherein a mass ratio of a content of the component (B) to a content of the component (C), i.e., the content of the component (B)/the content of the component (C), is 0.3 to 4.5.

6. The hydrogel particles of any one of claims 1-5, wherein the component (D) is agar.

7. The hydrogel particles of any one of claims 1-6, wherein the hydrogel particles has a continuous phase of hydrogel including the component (D) and the component (E) and a dispersed phase dispersed in the continuous phase and including the component (A), the component (B), and the component (C).

8. A dentifrice, comprising the hydrogel particles of any one of claims 1-7.

9. A cosmetic, comprising the hydrogel particles of any one of claims 1-7.

10. A method for producing hydrogel particles, comprising formation of droplets by dropping, spraying, or stirring a mixture including (A) a water-insoluble complex containing catechins, (B) a compound which forms chelate together with divalent or trivalent metal ions, (C) a divalent or trivalent metal salt, (D) a gel-forming agent, and (E) water.

11. The method of claim 10, comprising the step of forming (A) the water-insoluble complex containing catechins from (A1) catechins and (A2) polymer which forms the water-insoluble complex together with catechins.

12. The method of claim 11, wherein (A) the water-insoluble complex containing catechins is formed by mixing a first liquid containing (A2) the polymer which forms a water-insoluble complex together with catechins and a second liquid containing (A1) the catechins.

13. The method of claim 12, wherein an alkali is previously added to both or one of the first liquid and the second liquid before mixture of the first liquid and the second liquid.

14. The method of claim 13, wherein the alkali is previously added only to the first liquid before mixture with the second liquid.

15. The method of claim 14, wherein the first liquid to which the alkali is previously added has a pH of 6.0-9.0.

16. The method of any one of claims 13-15, wherein one of the first liquid or the second liquid includes (B) the compound which forms chelate together with divalent or trivalent metal ions, and the other liquid includes (C) the divalent or trivalent metal salt.

17. The method of any one of claims 10-16, wherein the droplets are further solidified by cooling.

18. The method of any one of claims 10-17, wherein the mixture is prepared by stirring and mixing with a stirring energy of 100 kW·min/m$^3$ or more.

## INTERNATIONAL SEARCH REPORT

International application No.

PCT/JP2010/006985

### A. CLASSIFICATION OF SUBJECT MATTER
*A61K8/49*(2006.01)i, *A61K8/02*(2006.01)i, *A61K8/365*(2006.01)i, *A61K8/73* (2006.01)i, *A61K8/81*(2006.01)i, *A61Q1/00*(2006.01)i, *A61Q11/00*(2006.01)i

According to International Patent Classification (IPC) or to both national classification and IPC

### B. FIELDS SEARCHED

Minimum documentation searched (classification system followed by classification symbols)
A61K8/49, A61K8/02, A61K8/365, A61K8/73, A61K8/81, A61Q1/00, A61Q11/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2009-196986 A (Kao Corp.),<br>03 September 2009 (03.09.2009),<br>entire text<br>(Family: none) | 1-18 |
| A | JP 2007-89579 A (Nitta Gelatin Inc.),<br>12 April 2007 (12.04.2007),<br>entire text<br>(Family: none) | 1-18 |

☐ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>24 February, 2011 (24.02.11) | Date of mailing of the international search report<br>08 March, 2011 (08.03.11) |
|---|---|
| Name and mailing address of the ISA/<br>Japanese Patent Office | Authorized officer |
| Facsimile No. | Telephone No. |

Form PCT/ISA/210 (second sheet) (July 2009)

| **INTERNATIONAL SEARCH REPORT** | International application No. |
|---|---|
| | PCT/JP2010/006985 |

<Subject of search>

In claims 1-10 and 17-18, a term "a catechin-containing water-insoluble complex" is described. However, it is unclear as to what substance is mixed with a catechin component to produce the water-insoluble complex, and the term can include great many combinations.

Those "catechin-containing water-insoluble complexes" disclosed in the meaning within PCT Article 5 are only "catechin-containing water-insoluble complexes" each produced by mixing a catechin component with a polymer represented by polymer A, and are therefore just some of the complexes included within the claimed scope. Therefore, it cannot be considered that the claims are supported by the disclosure of the description in the meaning within PCT Article 6.

Further, even though the common technical knowledge at the time of filing the present application is taken into consideration, it is impossible to specify the scope of the "catechin-containing water-insoluble complex". Therefore, claims 1-10 and 17-18 do not comply with the requirement of clarity under PCT Article 6, either.

Such being the case, the search was carried out only on "catechin-containing water-insoluble complexes" each produced by mixing a catechin component with a polymer represented by polymer A which are disclosed in the description specifically.

Form PCT/ISA/210 (extra sheet) (July 2009)

**EP 2 508 165 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008024651 A
- JP 2008024652 A
- JP 3972372 B
- JP 2007161683 A